# EUROPÄISCHE PATENTSCHRIFT

(11) **EP 0 882 507 B1**
(45) Veröffentlichungstag und Bekanntmachung des Hinweises auf die Patenterteilung: **30.10.2002**
(21) Anmeldenummer: 98109835.3
(22) Anmeldetag: 29.05.1998
(51) Int. Cl.: B01J 37/34, B01J 23/40, B01J 23/48, C07C 67/055

(54) **Verfahren zur Herstellung eines edelmetallhaltigen Trägerschalenkatalysators zur Herstellung von Vinylacetat**
Process for preparing a noble metal supported shell-type catalyst for the synthesis of vinyl acetate
Procédé de préparation d'un catalyseur supporté à base de métal précieux déposé en surface, ledit catalyseur étant utilisé pour la préparation d'acétate de vinyle

(30) Priorität: 05.06.1997 DE 19723591
(43) Veröffentlichungstag der Anmeldung: 09.12.1998
(73) Patentinhaber: Celanese Chemicals Europe GmbH, 60439 Frankfurt am Main (DE)
(72) Erfinder: Hagemeyer, Alfred, Dr., 65929 Frankfurt (DE); Dingerdissen, Uwe, Dr., 64342 Seeheim-Jugenheim (DE); Kühlein, Klaus, Prof. Dr., 65779 Kelkheim (DE); Heitz, Johannes, Dr., 4040 Linz (AT); Bäuerle, Dieter, Prof. Dr., 4203 Altenberg (AT)

(56) Entgegenhaltungen:
- US-A- 4 264 421
- US-A- 5 185 308
- US-A- 5 260 108

## Beschreibung

Die vorliegende Erfindung betrifft ein Verfahren zur Herstellung eines Katalysators durch UV-Photoreduktion von Metallsalzen auf einem Träger, den so hergestellten Katalysator, sowie seine Verwendung zur Herstellung von Vinylacetat.

Es ist bekannt, Vinylacetat in der Gasphase aus Ethylen, Essigsäure und Sauerstoff herzustellen. Die für diese Synthese verwendeten Trägerkatalysatoren enthalten Pd und ein Alkalimetall, vorzugsweise K. Als weitere Zusätze werden Cd, Au oder Ba verwendet. Die Metallsalze können durch Tränken, Aufsprühen, Aufdampfen, Imprägnieren, Tauchen oder Ausfällen auf den Träger aufgebracht werden.

Bei den Pd/Au/K-Katalysatoren hat es sich als vorteilhaft erwiesen, die beiden Edelmetalle in Form einer Schale auf den Träger aufzubringen, d.h. die Edelmetalle sind nur in einer oberflächennahen Zone verteilt, während die weiter innen liegenden Bereiche des Trägerformkörpers nahezu edelmetallfrei sind. Die Schichtdicke dieser katalytisch aktiven Schalen beträgt im allgemeinen ca. 0.1 - 2 mm. Mit Hilfe von Schalenkatalysatoren ist eine selektivere Verfahrensdurchführung möglich als mit Katalysatoren, bei denen die Trägerteilchen bis in den Kern imprägniert ("durchimprägniert") sind, bzw. eine Kapazitätserweiterung.
Dabei bietet es sich an, die Reaktionsbedingungen gegenüber den durchimprägnierten Katalysatoren unverändert zu halten und mehr Vinylacetat pro Reaktorvolumen und Zeit herzustellen. Dadurch wird die Aufarbeitung des erhaltenen Rohvinylacetats erleichtert, da der Vinylacetatgehalt im Reaktorausgangsgas höher ist, was weiterhin zu einer Energieersparnis im Aufarbeitungsteil führt. Geeignete Aufarbeitungen werden z.B. in US-5 066 365, DE-34 22 575, DE-34 08 239, DE-29 45 913, DE-26 10 624 und US-3 840 590 beschrieben. Hält man dagegen die Anlagenkapazität konstant, so kann man die Reaktionstemperatur senken und dadurch bei gleicher Gesamtleistung die Reaktion selektiver durchführen, wobei Edukte eingespart werden. Dabei wird auch die Menge des als Nebenprodukt entstehenden und daher auszuschleusenden Kohlendioxids und der mit dieser Ausschleusung verbundene Verlust an mitgeschlepptem Ethylen geringer. Darüberhinaus führt diese Fahrweise zu einer Verlängerung der Katalysatorstandzeit.

Eine Vielzahl von Dokumenten offenbart Katalysatoren und Verfahren zur Herstellung von Vinylacetat und Verfahren zu deren Herstellung. Es handelt sich dabei um durchimprägnierte Katalysatoren oder um Schalenkatalysatoren, die im allgemeinen zur Abscheidung der Edelmetalle auf dem Katalysatorträger einer chemischen Reduktion der auf den Träger aufgebrachten Edelmetallverbindungen unterworfen werden. Überraschenderweise wurde gefunden, daß die katalytisch aktiven Metalle auch durch Photoreduktion auf dem Träger abgeschieden werden können.

Die Abscheidung eines Metalls auf der Oberfläche eines Trägers kann aus einem Gas, einer Flüssigkeit oder einer adsorbierten Oberflächenschicht erfolgen. In den Druckschriften "Laser Processing and Chemistry" (Springer-Verlag, Berlin-Heidelberg-New York, 1996) und "Chemical Processing with Lasers" (Springer-Verlag, Berlin-Heidelberg-New York, 1986) von D. Bäuerle sind solche Verfahren für viele Metalle (darunter Ni, Ag, Au, Pd, Pt, Os und Ir) beschrieben.

Für das erfindungsgemäße Verfahren ist die Abscheidung von katalytisch aktiven Metallen aus adsorbierten Oberflächenschichten von besonderem Interesse.

W. Kräuter, D. Bäuerle, F. Fimberger, Appl. Phys. A 31, 13 (1983) beschreiben die laserinduzierte Abscheidung von Ni aus der Gasphase (Ni(CO)₄) mit einem Kryptonionenlaser mit Wellenlängen von 476 bis 647 nm. Als Substrat wurde Glas oder Si benutzt. Es wird darauf hingewiesen, daß der Beginn der Abscheidung auf die Photoreduktion einer adsorbierten Ni(CO)₄-Schicht zurückzuführen ist. Diese Photoreduktion ist mit Ultraviolett- (UV-) Licht wesentlich effizienter als mit sichtbarem Licht.

Y.-F. Lu, M. Takai, S. Nagatomo, K. Kato und S. Namba, Appl. Phys. A 54, 51 - 56 (1992) beschreiben die Abscheidung von Ag aus einer adsorbierten Ag-Acetatschicht auf einem Mn-Zn-Ferritsubstrat. Zur Bestrahlung wurde ein Argonionenlaser bei einer Wellenlänge von 514,5 nm verwendet.

R. C. Sausa, A. Gupta und J. R. White, J. Electrochem. Soc. 134, 2707 - 2713 (1987) beschreiben die Abscheidung von Pt auf Quarz aus einer metallorganischen Schicht ebenfalls durch Bestrahlung mit einem Argonionenlaser. Die Schicht wurde durch Verdampfen des Lösungsmittels aus einer Lösung hergestellt, die neben der metallorganischen Verbindung (Bright Platinum-05X, Engelhard Corporation) auch lackähnlichen Binder und Lösungsmittel enthielt. Das abgeschiedene Pt wurde als Keimschicht für die stromlose Kupferabscheidung eingesetzt.

H. Esrom, J. Demmy und U. Kogelschatz, Chemtronics 4, 202 - 208 (1989) berichten über den Einsatz einer Xe₂*-Excimerlampe (Wellenlänge 172 nm) zur Abscheidung von Pd-Keimen auf Aluminiumoxidsubstraten für die stromlose Kupferabscheidung. Als adsorbierte Schicht wurde Pd-Acetat verwendet.

Auch Y. Zhang und M. Stuke, Chemtronics 4, 212 - 215 (1989) beschreiben die Abscheidung von Pd aus einer Pd-Acetatschicht auf Aluminiumoxidkeramiken, Quarzsubstraten und Siliziumwafern. Zur Bestrahlung wurde das Synchrotronlicht eines Elektronenspeicherringes im Wellenlängenbereich von 40 - 400 nm eingesetzt.

H. Esrom und G. Wahl, Chemtronics 4, 216 - 223 (1989) beschreiben die Photoreduktion von Pd-Acetat durch die Bestrahlung mit Licht aus einem ArF-Excimerlaser (Wellenlänge 193 nm) und einem KrF-Excimerlaser (Wellenlänge 248 nm). Mit diesem Verfahren wurden Pd-Keime für die stromlose Kupferabscheidung auf Quarz und Aluminiumoxidkeramiken abgeschieden.

A.G. Schrott, B. Braren und R. Saraaf, Appl. Phys. Lett. 64, 1582 - 1584 (1994) berichten über die Photoreduktion von PdSO₄ zu metallischem Pd mit einem Excimerlaser. Auch hier konnte gezeigt werden, daß bekeimte Substrate (SiO₂) für die stromlose Kupferabscheidung benutzt werden konnten.

P.B. Comita, E. Kay, R. Zhang und W. Jacob, Appl. Surf. Sci. 79/80, 196 - 202 (1994) beschreiben die laserinduzierte Koaleszenz von Goldclustern in einer dünnen Fluorkohlenstoffschicht, die durch Plasmapolymerisation hergestellt wurde. Bei der Herstellung dieser Schicht wurde durch Ionensputtern Gold eingelagert. Durch Bestrahlung mit einem Argonionenlaser wurde die Polymermatrix aufgebrochen und verdampft und es bleiben zusammenhängende Goldstrukturen zurück.

Keine dieser Druckschriften offenbart ein Verfahren zur Herstellung von Katalysatoren.

US-4,264,421 offenbart jedoch ein Verfahren zur Herstellung von mit Edelmetallen beschichteten Pulvern, beispielsweise TiO₂ oder WO₃, bei dem eine Suspension dieser Pulver in einer Metallsalzlösung, beispielsweise in einer Platinsalzlösung, bestrahlt wird, wobei es dann zu einer einheitlichen Abscheidung der Edelmetalle auf dem Pulver kommt. Anschließend wird das mit Metallen beladene Pulver abgetrennt, mit Wasser gewaschen und getrocknet. Es kann als Katalysator in einer heterogenen photokatalytischen Decarboxylierung von Carbonsäuren eingesetzt werden.

Zur photoinduzierten Abscheidung von Edelmetallen aus adsorbierten Oberflächenschichten werden sowohl UV-Lichtquellen als auch Lichtquellen, die sichtbares Licht aussenden, eingesetzt. Da die Absorptionskoeffizienten der im erfindungsgemäßen Verfahren verwendeten Stoffe im ultravioletten Spektralbereich wesentlich höher sind als im sichtbaren Spektralbereich, kann mit entsprechend niedrigeren Leistungsdichten gearbeitet werden, falls UV-Lichtquellen eingesetzt werden. Da dadurch ein wesentlich höherer Durchsatz erreicht wird, ist der Einsatz von UV-Lichtquellen bevorzugt, wobei die Quellen mit den kürzesten Wellenlängen im allgemeinen die höchste Effizienz aufweisen, und daher deren Verwendung besonders bevorzugt ist.

Die zur Photoreduktion verwendeten UV-Stahlungsquellen sind Stand der Technik. Es handelt sich dabei um Lampen, Laser oder sonstige Strahlungsquellen wie Elektronenspeicherringe (Sychrotrons) oder Plasmaentladungen. Bei der Benutzung von Lampen stehen vor allem Hg-Dampflampen (mit starken Emissionslinien bei Wellenlängen von 185 nm und 254 nm) und spektral eng begrenzte Excimerlampen, bei denen die UV-Strahlung durch den Zerfall von Excimeren oder Exciplexen wie Kr₂* (Wellenlänge 146 nm), Xe₂* (172 nm), KrCl* (222 nm) oder XeCl* (308 nm) entsteht, zur Verfügung. Als leistungsstarke UV-Laser werden gepulste Excimerlaser eingesetzt. Auch hier entsteht das Licht durch den Zerfall von Excimeren oder Exciplexen wie F₂* (157 nm), ArF* (193 nm), KrF* (248 nm), XeCl* (308 nm) und XeF* (351 nm). Es können auch frequenzvervielfachte Nd-YAG:Laser (Wellenlänge 1064 nm / n = 3,4,5, .....) verwendet werden. Weitere Quellen für UV-Strahlung sind Sychrotrons, die eine breitbandige Strahlung bis in den Röntgenbereich liefern, und das Licht einer Plasmaentladung bei niedrigem Druck.

Aufgabe der vorliegenden Erfindung war, ein Verfahren zur Herstellung edelmetallhaltiger Schalenkatalysatoren bereitzustellen, das auf chemische Reduktionsmittel verzichtet und eine einfache Einstellbarkeit der Schalendicke erlaubt. Ferner war es eine Aufgabe der vorliegenden Erfindung, einen aktiven und selektiven Vinylacetat-Schalenkatalysator auf Basis Pd/Au mit wenigen Arbeitsschritten, schnell und kostengünstig herzustellen, und dabei eine leichte Steuerbarkeit der Schalendicke zu ermöglichen.

Die gestellten Aufgaben werden durch die Erfindung gelöst, indem trägerfixierte Edelmetallsalze durch Photoreduktion mittels UV-Strahlung in einer äußeren Schale des Trägerformkörpers zum Metall reduziert und fixiert werden. Die Schalendicke kann über die Eindringtiefe der UV-Strahlung eingestellt werden. Auf diese Weise wird eine gute Einheitlichkeit der katalytisch aktiven Metallteilchen, eine schmalbandige Partikelgrößenverteilung und eine hohe Metalldispersion in der Schale erreicht.

Gegenstand der vorliegenden Erfindung ist ein Verfahren zur Herstellung von edelmetallhaltigen Schalenkatalysatoren auf einem porösen Träger, wobei der Träger mit Salzlösungen der Edelmetalle imprägniert, der Katalysator-Vorläufer danach getrocknet und anschließend einer UV-Strahlung ausgesetzt wird, so daß die Edelmetallsalze in der oberflächennahen Zone zu den Metallen reduziert werden.

Die Photoreduktion erfolgt vorzugsweise mit einer monochromatischen UV-Excimer-Strahlung. Vorzugsweise wird in Abwesenheit von chemischen Reduktionsmitteln gearbeitet.

Die nichtbestrahlten und daher nicht reduzierten Metallsalze im Korninneren der Pellets werden nach der Bestrahlung des Trägers mit einem Lösungsmittel extrahiert, wobei die in der Schale fixierten Edelmetall-Nanoteilchen aufgrund ihrer Unlöslichkeit nicht ausgewaschen werden und ortsfest bleiben.

Die auf diese Weise hergestellten Schalenkatalysatoren können für eine Vielzahl von heterogen katalysierten Reaktionen wie Hydrierungen und Oxidationen eingesetzt werden.

Nach diesem Verfahren hergestellte Pd/Au-Schalenkatalysatoren sind zur Verwendung in der Vinylacetat-Synthese geeignet. Im Vergleich zu konventionellen Präparationstechniken von Edelmetall-Trägerkatalysatoren (Imprägnierung mit Metallsalzen und deren chemische Reduktion) kann bei der erfindungsgemäßen Photoreduktion auf das chemische Reduktionsmittel und auf die damit verbundenen Nachteile wie Kontamination des Trägers mit Fremdmetallen, Entsorgung des Salzanfalls, Mehrstufigkeit und energie- und zeitintensive Handhabung von oft giftigen Lösungen verzichtet werden.

Gegenüber den konventionellen Verfahren zur Schalenerzeugung (Fixierung durch Basenfällung gefolgt von chemischer Reduktion) hat das erfindungsgemäße Verfahren den Vorteil einer leichten Steuerbarkeit und Kontrollierbarkeit der Schalendicke über die für die Abscheidung wesentlichen physikalischen Parameter wie Wellenlänge und Leistung der UV-Strahlungsquelle, sowie Konzentration der Imprägnierlösung, und Zeit und Temperatur der Photoreduktion. Die Reduktion und Fixierung in der Schale erfolgen beim erfindungsgemäßen Verfahren gleichzeitig in einem Schritt.

Bevorzugt sind solche Katalysatoren mit einer Schalendicke von 5 bis 5000 µm. Weiterhin sind solche Katalysatoren bevorzugt, die Pd und/oder Au enthalten.

Die erfindungsgemäße Photoreduktion dauert nur wenige Minuten, während für die herkömmliche Basenfixierung ca. 20 h gebraucht werden.

Die erfindungsgemäß hergestellten Schalenkatalysatoren zeichnen sich aufgrund der genannten Eigenschaften durch hohe Aktivitäten und Selektivitäten aus. Als Aktivmetalle, die in der Schale konzentriert werden können, sind alle Metalle geeignet, für die photoreduzierbare Vorläufer existieren. Voraussetzung dafür ist eine ausreichende UV-Absorption der Vorläufer bei der eingestrahlten Wellenlänge. Durch geeignete Auswahl der zur Bestrahlung eingesetzten Wellenlänge kann für eine Vielzahl von einfachen und verfügbaren Metallsalzen wie z.B. Acetaten, Formiaten, Propionaten, Butyraten, Nitraten, Sulfaten oder Chloriden eine UV-Absorption erreicht werden. Auch können die imprägnierten Träger vor der Bestrahlung mit UV-Licht mit Sensibilisatoren beaufschlagt werden. Aufgrund der leichten Photoreduzierbarkeit sind insbesondere alle Edelmetalle sowie deren Mischungen, geeignet. Bevorzugt sind Pd, Au, Pt, Ag, Rh, Ru, Os und Ir. Besonders bevorzugt sind Pd und Au.

Als Träger werden inerte Materialien, wie SiO₂, Al₂O₃, TiO₂, ZrO₂ oder Gemische dieser Oxide in Form von Kugeln, Tabletten, Ringen, Sternen oder anderen Formkörpern benutzt. Der Durchmesser bzw. die Länge und Dicke der Trägerteilchen liegt im allgemeinen bei 3 bis 9 mm. Die Oberfläche der Träger liegt, gemessen mit der BET-Methode, im allgemeinen bei 10 - 500 m²/g, bevorzugt bei 20 - 250 m²/g. Das Porenvolumen liegt im allgemeinen bei 0.3 bis 1.2 ml/g.

Die Reduktion und Trägerfixierung der Edelmetallvorläufer erfolgt erfindungsgemäß durch UV-Licht. Dabei können beispielsweise folgende UV-Strahlungsquellen verwendet werden: UV-Excimerlaser, frequenzvervielfachte Nd:YAG-Laser, UV-Excimeriampen, Hg-Dampflampen, Synchrotrons oder Niederdruckplasmaentladungen. Bevorzugt kommt UV-Excimerstrahlung zum Einsatz, die monochromatisch ist und hohe Leistungsspitzen aufweist. Geeignete Wellenlängen liegen im Bereich von 40 bis 400 nm. Bevorzugte Wellenlängen liegen zwischen 140 und 360 nm, insbesondere 172 nm (Xe₂*-Lampe), 193 nm (ArF*-Laser), 222 nm (KrCl*-Lampe), 248 nm (KrF*-Laser) und 308 nm (XeCl*-Lampe). Bevorzugte UV-Leistungsdichten liegen zwischen 0,01 und 100 W/cm², besonders bevorzugte UV-Leistungsdichten zwischen 0,1 und 20 W/cm².

Bei Einsatz gepulster Laser liegen die geeigneten Pulsfrequenzen im allgemeinen im Bereich zwischen 0,1 und 5000 Pulse/s. Die Einstrahlzeiten liegen im allgemeinen zwischen 0,01 s und 3600 s. Bevorzugte Pulsfrequenzen sind 1 bis 1000 Pulse/s und bevorzugte Einstrahlzeiten 0,01 bis 1000 s, insbesondere 0,1 bis 300 s.

Beim Einsatz von UV-Lampen, die nicht gepulst sind und wesentlich größere räumliche und spektrale Bestrahlungsfenster als UV-Laser aufweisen können, liegen die geeigneten Bestrahlungszeiten zwischen 1s und 10 h. Bevorzugte Bestrahlungszeiten liegen zwischen 0,1 min und 100 min.

Durch die begrenzte Eindringtiefe der UV-Strahlung kann die Schalendicke leicht eingestellt und gesteuert werden.

Sollen mehrere Edelmetalle auf dem Träger fixiert werden (z.B. Pd und Au), so können diese bei geeigneter Wahl der für die Abscheidung wesentlichen physikalischen Parameter nach dem erfindungsgemäßen Verfahren gleichzeitig photoreduziert werden, wobei im allgemeinen Legierungsteilchen entstehen. Alternativ kann auch eine sequentielle Photoreduktion unter jeweils für die Einzelmetalle optimierten Einstrahlbedingungen durchgeführt werden, die zu strukturierten Edelmetallteilchen führen kann.

Die Photoreduktion kann auch mit der konventionellen chemischen Reduktion kombiniert werden. Beispielsweise kann die Photoreduktion nur zur Vorbekeimung in der Schale eingesetzt werden, die dann durch erneute Tränkung mit gleichen oder anderen Metallsalzen und deren chemische Reduktion verstärkt wird. Ebenso kann auch nur eines der beiden Metalle photoreduziert werden und das andere Metall danach mit konventionellen Methoden aufgebracht und reduziert werden. Beispielsweise kann zuerst mit Pd-Acetat imprägniert werden, das dann in einer Schale zu Pd-Metall photoreduziert wird. Anschließend kann mit Au-Salzen nachgetränkt werden, deren Reduktion naßchemisch oder auch in-situ im Reaktor mit gasförmigen Reduktionsmitteln wie H₂ oder Ethylen erfolgen kann. Auf diese Weise wird sichergestellt, daß das eigentliche Aktivmetall für den Vinylacetat-Prozeß, also das Pd, in einer Schale fixiert wird, während die Verteilung des Aktivators, also des Au, weniger kritisch ist und der daher auch mit konventionellen chemischen Verfahren fixiert werden kann.

Die nicht bestrahlten und daher nicht reduzierten Edelmetallsalze im Korninneren der Pellets werden mit einem Lösungsmittel extrahiert. Geeignete Lösungsmittel sind z.B. Chloroform, Essigsäure oder wäßrige Lösungen von Zitronensäure oder Oxalsäure

Das erfindungsgemäße Verfahren eignet sich insbesondere zur Herstellung von Vinylacetat-Schalenkatalysatoren. Von diesen gibt es 3 Arten, die sich im wesentlichen aus Pd/Cd/K, Pd/Ba/K oder Pd/Au/K zusammensetzen. Die fertigen Vinylacetat-Katalysatoren weisen folgende Zusammensetzungen auf:

Der Pd-Gehalt der Pd/K/Cd- und der Pd/K/Bs-Katalysatoren beträgt im allgemeinen 0.6 bis 3.5 Gew.-%, vorzugsweise 0.8 bis 3.0 Gew.-%, insbesondere 1.0 bis 2.5 Gew.-%. Der Pd-Gehalt der Pd/Au/K-Katalysatoren beträgt im allgemeinen 0.5 bis 2.0 Gew.-%, vorzugsweise 0.6 bis 1.5 Gew.-%.
Der K-Gehalt aller drei Katalysator-Arten beträgt im allgemeinen 0.5 bis 4.0 Gew.-%, vorzugsweise 1.5 bis 3.0 Gew.-%.
Der Cd-Gehalt der Pd/K/Cd-Katalysatoren beträgt im allgemeinen 0.1 bis 2.5 Gew.-%, vorzugsweise 0.4 bis 2.0 Gew.-%.
Der Ba-Gehalt der Pd/K/Ba-Katalysatoren beträgt im allgemeinen 0.1 bis 2.0 Gew.-%, vorzugsweise 0.2 bis 1.0 Gew.-%.
Der Au-Gehalt der Pd/K/Au-Katalysatoren beträgt im allgemeinen 0.2 bis 1.0 Gew.-%, vorzugsweise 0.3 bis 0.8 Gew.-%.

Als Salze sind alle Salze von Palladium, Cadmium, Barium, Gold und Kalium geeignet, die löslich sind und keine für den Katalysator giftigen Bestandteile, wie z.B. Schwefel enthalten. Bevorzugt sind die Acetate und die Chloride. Dabei muß aber im Falle der Chloride sichergestellt werden, daß die Chloridionen vor dem Einsatz des Katalysators entfernt werden. Dies geschieht durch Auswaschen des dotierten Trägers, z.B. mit Wasser, nachdem Pd und ggf. Au durch Reduktion zu den Metallpartikeln auf dem Träger fixiert wurden.

Als Lösungsmittel zur Imprägnierung sind alle Verbindungen geeignet, in denen die gewählten Salze löslich sind und die nach der Imprägnierung leicht wieder durch Trocknung zu entfernen sind. Geeignet sind für die Acetate vor allem unsubstituierte Carbonsäuren, insbesondere Essigsäure. Für die Chloride ist vor allem Wasser geeignet. Die zusätzliche Verwendung eines weiteren Lösungsmittels ist dann zweckmäßig, wenn die Salze in der Essigsäure oder im Wasser nicht genügend löslich sind. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, Acetonitril, Dimethylformamid, aber auch Kohlenwasserstoffe wie Benzol.

Von jedem der auf die Trägerteilchen aufzubringenden Elemente (Pd/K/Au, Pd/K/Cd, Pd/K/Ba) wird im allgemeinen mindestens ein Salz aufgebracht. Man kann mehrere Salze eines Elements aufbringen, aber im allgemeinen bringt man von jedem der drei Elemente genau ein Salz auf. Die notwendigen Salzmengen können in einem Schritt oder durch Mehrfachimprägnierung aufgebracht werden. Die Salze können nach bekannten Verfahren wie Tränken, Aufsprühen, Aufdampfen, Tauchen, Imprägnieren oder Ausfällen auf den Träger aufgebracht werden.

Nach dem erfindungsgemäßen Verfahren werden nur die Edelmetallsalze, also Pdund Au-Salze, zu den entsprechenden Edelmetall-Nanoteilchen reduziert und nicht die "unedlen" Bestandteile K, Cd, Ba. Letztere können zusammen mit den Edelmetallsalzen oder auch vorher oder nachher auf den Träger aufgebracht werden. Normalerweise wird nach dem erfindungsgemäßen Verfahren zuerst eine Schale aus Pd/Au erzeugt und danach mit K-Acetat-Lösung nachgetränkt, wobei das K gleichmäßig über den Pelletquerschnitt verteilt ist.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Die folgenden Beispiele sollen die Erfindung erläutern.

### Beispiel 1:

a) Imprägnierung von porösen SiO₂-Tabletten mit Pd-Acetat:
   50 ml Aerosil200-Tabletten (5.5 x 6 mm, Degussa) werden vorgelegt (ca. 25 g). 530 mg Pd-Acetat (Aldrich) wird in 30 ml Eisessig gelöst (entspricht 1 Gew.-% Pd). Die Lösung wird über ein Faltenfilter filtriert. Die klare Pd-Lösung wird zu den Aerosil-Tabletten gegeben und der Eisessig am Rotavapor über 2 h unter ständigem Drehen wieder abgezogen. Anschließend werden noch vorhandene Essigreste im Ölpumpenvakuum bei 0.2 mbar / 60°C abgezogen.
b) Photoreduktion:
   Die Stirnflächen der Tabletten wurden mit einem KrF*-Laser (Wellenlänge 248 nm) in Luft mit je 500 Laserpulsen bestrahlt. Die Energiedichte des Laserlichts auf der Probenoberfläche betrug 350 mJ/cm². Die Pulsfrequenz des Lasers war 10 Pulse/s. Nach Durchschneiden einer repräsentativen Anzahl von Pellets wurde die Schalendicke mittels Lichtmikroskopie und XPS-Line-Scans ausgemessen. Die Schalendicke beträgt ca. 0.5 mm.
c) Konfektionierung zum technischen Katalysator:
   20ml bestrahlte Aerosil - Tabletten werden mit 2l Essigsäure, 40% + 10% Kaliumacetat, im Soxhlet gewaschen und bei 110°C getrocknet.
   Da die Tabletten bereits 1% Pd enthalten, wird hier nur noch Au aufgetragen: 125,4mg Au(CH₃COO)₃ (entspr. 66mg Au), hergestellt nach der Vorschrift aus US-4 933 204, werden in 10ml H₂O gelöst und zu den Tabletten gegeben. Am Rotavapor läßt man unter Drehen und unter einem N₂-Strom eintrocknen. Danach wird bei 110°C getrocknet. 0,8g Kaliumacetat werden in 15ml H₂O gelöst und w.o. auf die Tabletten verbracht, bei 110°C für 4h getrocknet, danach noch zusätzlich im Vakuum über Nacht nachgetrocknet.
d) Reaktortests
   Reaktortests für die Gasphasenoxidation von Ethylen und Essigsäure zu Vinylacetat:
   Die Katalysatoren werden in einem Festbett-Rohrreaktor mit 2 cm Rohrduchmesser getestet. Der Reaktor wird von außen mit einer Öl-Mantelheizung temperiert. Es werden 15 ml der Katalysator-Formkörper vorgelegt. Das Reaktorvolumen vor und nach der Katalysatorschüttung wird mit Glaskugeln aufgefüllt. Die Testapparatur wird von einem Prozeßleitsystem gesteuert und kontinuierlich betrieben. Der Katalysator wird zunächst aktiviert und dann unter konstanten Reaktionsbedingungen getestet.

Die Aktivierung besteht aus mehreren Schritten: Aufheizen unter N₂, Zugabe von Ethylen, Druckerhöhung, Zugabe Essigsäure, Halten der Bedingungen, Zugabe Sauerstoff.

Die Reaktionsbedingungen bei den Tests sind 160-170°C Reaktionstemperatur, 8-9 bar Überdruck. Der Feed setzt sich zusammen aus 64.5 Vol.-% Ethylen, 16.1 Vol.-% N₂, 14.3 Vol.-% Essigsäure und 5.1 Vol.-% O₂. Eine Vollanalyse des Reaktoraustrags wird direkt am Reaktorausgang mittels on-line GC (2 Säulen-Schaltung) durchgeführt.

Die Reaktorergebnisse finden sich in der nachfolgenden Tabelle. Die Konzentrationsverhältnisse der beteiligten Stoffe sind in GC-Flächenprozenten angegeben.

**Tabelle 1**

| Probe | T (°C) | p (bar) | GC-Analyse des Reaktoraustrags | | | | | | |
|---|---|---|---|---|---|---|---|---|---|
| | | | CO₂ | C₂H₄ | O₂ | N₂ | H₂O | Vinylacetat | Essigsäure |
| Beispiel 1 | 160 | 9 | 0.85 | 55.7 | 3.25 | 19.5 | 0.8 | 1.27 | 18.5 |
| Beispiel 1 | 170 | 9 | 1.4 | 53.8 | 2.59 | 23.1 | 1.18 | 1.15 | 16.8 |

### Beispiel 2:

a) Imprägnierung von porösen SiO₂-Tabletten mit Pd-Acetat und Au-Acetat:
50 ml (ca. 25 g) Aerosil200-Tabletten (5.5 x 6 mm, Degussa) werden vorgelegt. 530 mg Pd-Acetat (Aldrich) (entspricht 1 Gew.-% Pd) wird in 30 ml Eisessig gelöst. 290 mg Au-Acetat (entspricht 0.6 Gew.-% Au), hergestellt nach der Vorschrift aus US 4 933 204 werden in 10 ml Eisessig gelöst. Die beiden Lösungen werden zusammengegeben und über ein Faltenfilter filtriert. Die klare Pd-Au-Lösung wird zu den Aerosil-Tabletten gegeben und der Eisessig am Rotavapor über 2 h unter ständigem Drehen wieder abgezogen. Anschließend werden noch vorhandene Essigreste im Ölpumpenvakuum bei 0.2 mbar / 60°C abgezogen. Das Endgewicht der imprägnierten Tabletten beträgt 25,8 g.
b) Photoreduktion:
Die imprägnierten Tabletten wurden mit einem KrF*-Laser (Wellenlänge 248 nm) in Luft mit je 150 Laserpulsen auf beiden Stirnflächen bestrahlt. Die Energiedichte (Fluenz) des Laserlichts auf der Probenoberfläche betrug 350 mJ/cm². Die Pulsfrequenz des Lasers war 10 Pulse/s.
Nach Durchschneiden einer repräsentativen Anzahl von Pellets wurde die Schalendicke mittels Lichtmikroskopie und XPS-Line-Scans ausgemessen. Die Anzahl der Pulse ist so gewählt, daß die Schalendicke ca. 0.9 mm beträgt.
Der durch die Bestrahlung induzierte Farbumschlag von gelb nach schwarzbraun war deutlich zu erkennen. Im Gegensatz zu Beispiel 1 (Vortränkung nur mit Pd-Acetat) war es bei der Pd-Au-Vorimprägnierung leichter, einen Farbumschlag zu erreichen.
c) Konfektionierung zum technischen Katalysator
Die bestrahlten Tabletten werden im Soxhlet zunächst mit 2000ml 40% Essigsäure, danach mit 1000ml Wasser gewaschen, über Nacht bei 110°C und Normaldruck getrocknet, danach noch 1 Stunde im Vacuum. 2g Kaliumacetat werden in 30ml Wasser gelöst und auf einmal zu den Pellets gegeben. Es wird für 15 min unter ständigem Drehen gemischt und wieder bei 110°C und Normaldruck getrocknet, zuletzt noch 1 Stunde unter Vacuum.
d) Reaktortests
Die Herstellung von Vinylacetat erfolgte unter den gleichen Bedingungen wie unter d) in Beispiel 1 angegeben. Die Reaktorergebnisse finden sich in der nachfolgenden Tabelle. Die Konzentrationsverhältnisse der beteiligten Stoffe sind in GC-Flächenprozenten angegeben.:

**Tabelle 2:**

| Probe | T (°C) | p (bar) | GC-Analyse des Reaktoraustrags | | | | | | |
|---|---|---|---|---|---|---|---|---|---|
| | | | CO₂ | C₂H₄ | O₂ | N₂ | H₂O | Vinylacetat | Essigsäure |
| Beispiel 2 | 160 | 9 | 0.01 | 56.8 | 4.3 | 19.5 | 0.06 | 0.34 | 19.0 |

## Patentansprüche

1. Verfahren zur Herstellung von edelmetallhaltigen Schalenkatalysatoren auf einem porösen Träger, wobei der Träger mit Salzlösungen der Edelmetalle imprägniert, der Katalysator-Vorläufer danach getrocknet und anschließend einer UV-Strahlung ausgesetzt wird, so daß die Edelmetallsalze in der oberflächennahen Zone zu den Metallen reduziert werden, und anschließend bei erhöhter Temperatur mit einem Lösungsmittel extrahiert wird.

2. Verfahren nach Anspruch 1, **dadurch gekennzeichnet, daß** die Edelmetalle ausgewählt sind aus der Gruppe bestehend aus Pd, Au, Pt, Ag, Rh, Ru, Os und Ir.

3. Verfahren nach Anspruch 1 und/oder 2, **dadurch gekennzeichnet, daß** die Edelmetalle Au und Pd sind, und als deren Salze die Acetate verwendet werden.

4. Verfahren nach einem oder mehreren der Ansprüche 1 bis 3, **dadurch gekennzeichnet, daß** als Trägermaterialien SiO₂, Al₂O₃, TiO₂, ZrO₂ oder deren Mischungen verwendet werden.

5. Verfahren nach einem oder mehreren der Ansprüche 1 bis 4, **dadurch gekennzeichnet, daß** die UV-Photoreduktion durch eine Bestrahlung mit Licht einer Wellenlänge von 40 bis 400 nm, insbesondere von 140 bis 360 nm durchgeführt wird.

6. Verfahren nach einem oder mehreren der Ansprüche 1 bis 5, **dadurch gekennzeichnet, daß** die Photoreduktion des Pd und/oder Au durch eine Bestrahlung mit UV-Licht mit einer Leistungsdichte von 0,01 bis 100 W/cm², insbesondere 0,1 bis 20 W/cm², aus einer Lampe oder einem gepulsten Laser durchgeführt wird, wobei bei Verwendung eines gepulsten Lasers die Pulsfrequenz zwischen 1 und 1000 Pulse/s und die Bestrahlungszeit zwischen 0,01 und 1000 s liegt, während bei Verwendung einer Lampe die Bestrahlungszeit zwischen 0,1 min und 100 min liegt.

7. Verfahren nach einem oder mehreren der Ansprüche 1 bis 6, **dadurch gekennzeichnet, daß** der imprägnierte Katalysatorträger vor der Bestrahlung mit UV-Sensibilisatoren beaufschlagt wird.

8. Verfahren nach einem oder mehreren der Ansprüche 1 bis 7, **dadurch gekennzeichnet, daß** ohne ein chemisches Reduktionsmittel gearbeitet wird.

## Claims

1. A process for producing shell catalysts comprising noble metals on a porous support, which comprises impregnating the support with salt solutions of the nobel metals, drying the catalyst-precursor and subsequently exposing it to UV radiation so that the noble metal salts in the zone close to the surface are reduced to the metals, and subsequently extracting with a solvent at elevated temperature.

2. The process as claimed in claim 1, wherein the noble metals are selected from the group consisting of Pd, Au, Pt, Ag, Rh, Ru, Os and Ir.

3. The process as claimed in claim 1 and/or 2, wherein the noble metals are Au and Pd and the salts thereof which are used are the acetates.

4. The process as claimed in one or more of claims 1 to 3, wherein the support material used is SiO₂, Al₂O₃, TiO₂, ZrO₂ or a mixture thereof.

5. The process as claimed in one or more of claims 1 to 4, wherein the UV photoreduction is carried out by irradiation with light having a wavelength of from 40 to 400 nm, in particular from 140 to 360 nm.

6. The process as claimed in one or more of claims 1 to 5, wherein the photoreduction of the Pd and/or Au is carried out by irradiation with UV light having a power density of from 0.01 to 100 W/cm², in particular from 0.1 to 20 W/cm², from a lamp or a pulsed laser, where, when a pulsed laser is used, the pulse frequency is from 1 to 1000 pulses/s and the irradiation time is from 0.01 to 1000 s, while when a lamp is used the irradiation time is from 0.1 min to 100 min.

7. The process as claimed in one or more of claims 1 to 6, wherein the impregnated catalyst support is treated with UV sensitizers before irradiation.

8. The process as claimed in one or more of claims 1 to 7 carried out without using a chemical reducing agent.

## Revendications

1. Procédé pour la préparation de catalyseurs à enveloppes contenant des métaux nobles sur un support poreux dans lequel on imprègne le support par des solutions de sels des métaux nobles, on sèche ensuite ce produit intermédiaire de catalyseur puis on l'expose à un rayonnement UV qui réduit les sels de métaux nobles en métaux dans la zone voisine de la surface, après quoi on extrait par un solvant à haute température.

2. Procédé selon la revendication 1, **caractérisé en ce que** les métaux nobles sont choisis dans le groupe consistant en Pd, Au, Pt, Ag, Rh, Ru, Os et Ir.

3. Procédé selon la revendication 1 et ou 2, **caractérisé en ce que** les métaux nobles consistent en Au et Pd, et **en ce que** les sels utilisés sont leurs acétates.

4. Procédé selon une ou plusieurs des revendications 1 à 3, **caractérisé en ce que** les matières de support consistent en SiO₂, Al₂O₃, TiO₂, ZrO₂ ou leurs mélanges.

5. Procédé selon une ou plusieurs des revendications 1 à 4, **caractérisé en ce que** la photoréduction par UV est réalisée par irradiation à une lumière de longueur d'onde 40 à 400 nm, plus spécialement 140 à 300 nm.

6. Procédé selon une ou plusieurs des revendications 1 à 5, **caractérisé en ce que** la photoréduction de Pd et/ou de Au est réalisée par irradiation à la lumière UV d'une puissance de 0,01 à 100 W/cm², plus spécialement de 0,1 à 20 W/cm², provenant d'une lampe ou d'un laser pulsé, la fréquence de pulsation dans ce dernier cas se situant entre 1 et 1000 pulsations/s avec une durée d'irradiation de 0,1 à 1000 s, alors que dans le cas où on utilise une lampe, la durée d'irradiation est de 0,1 min à 100 min.

7. Procédé selon une ou plusieurs des revendications 1 à 6, **caractérisé en ce que** sur le support de catalyseur imprégné et avant l'exposition aux UV, on applique des sensibilisants.

8. Procédé selon une ou plusieurs des revendications 1 à 7, **caractérisé en ce que** l'on opère en l'absence d'agent réducteur chimique.
